(19) Europäisches Patentamt
European Patent Office
Office européen des brevets

(11) EP 2 725 012 A1

(12) **EUROPEAN PATENT APPLICATION**
published in accordance with Art. 153(4) EPC

(43) Date of publication:
**30.04.2014 Bulletin 2014/18**

(21) Application number: **12802722.4**

(22) Date of filing: **18.06.2012**

(51) Int Cl.:
***C07C 237/24*** *(2006.01)* ***C07C 227/18*** *(2006.01)*
***C07C 227/30*** *(2006.01)* ***C07C 229/48*** *(2006.01)*
***C07C 231/06*** *(2006.01)* ***C07C 231/16*** *(2006.01)*
***C12P 13/02*** *(2006.01)* ***C07B 53/00*** *(2006.01)*

(86) International application number:
**PCT/JP2012/065452**

(87) International publication number:
**WO 2012/176715 (27.12.2012 Gazette 2012/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2011 JP 2011137246**

(71) Applicant: **Mitsubishi Gas Chemical Company, Inc.**
**Tokyo 100-8324 (JP)**

(72) Inventor: **MATSUMOTO, Masahiro**
**Niigata-shi**
**Niigata 950-3112 (JP)**

(74) Representative: **Müller-Boré & Partner Patentanwälte PartG mbB**
**Friedenheimer Brücke 21**
**80639 München (DE)**

(54) **1-AMINO-2-VINYL CYCLOPROPANE CARBOXYLIC ACID AMIDE, SALT OF SAME, AND METHOD FOR PRODUCING SAME**

(57) The present invention relates to 1-amino-2-vinylcyclopropane carboxylic acid amide or a salt thereof. By obtaining optically active 1-amino-2-vinylcyclopropane carboxylic acid amide or a salt thereof by hydrolyzing optically active 1-amino-2-vinylcyclopropane carbonitrile or a salt thereof according to the production method of the present invention, 1-amino-2-vinylcyclopropane carboxylic acid amide or a salt thereof, which is useful as a pharmaceutical/agrochemical intermediate, can be easily obtained. The present invention is capable of providing a substrate to be subjected to optical resolution, which enables the production of optically active 1-amino-2-vinylcyclopropane carboxylic acid, which is widely used as a raw material for pharmaceutical and agrochemical products and is especially important as an intermediate for therapeutic agents for hepatitis C, inexpensively with high purity and high yield.

EP 2 725 012 A1

**Description**

CROSS-REFERENCE TO RELATED APPLICATION

This application is based on a preceding Japanese Patent Application No. 2011-137246 (filed on June 21, 2011), which claims the benefit of the priority and the entirety of the disclosure of which is incorporated herein by reference.

TECHNICAL FIELD

The present invention relates to 1-amino-2-vinylcyclopropane carboxylic acid amide and a salt thereof, which are very useful as pharmaceutical and agrochemical intermediates.

BACKGROUND ART

1-Amino-2-vinylcyclopropane carboxylic acid is widely used as a raw material for pharmaceutical and agrochemical products. In particular, optically active 1-amino-2-vinylcyclopropane carboxylic acid is very important as a pharmaceutical product, especially as an intermediate for therapeutic agents for hepatitis C.

1-Amino-cyclopropane carboxylic acid derivative is known to function as an inhibitor of pyridoxal-phosphate dependent enzymes and attracts attention as a lead compound for antibiotic drugs, antiviral agents, antitumor drugs and the like, especially as a candidate compound for development of pharmaceutical products.

Since it has recently been found that the Hepatitis C virus NS3/4A protease can be inhibited by a compound which has 1-amino-2-vinylcyclopropane carboxylic acid having a vinyl group at position 2 of 1-amino-cyclopropane carboxylic acid as a part of the basic skeleton and that the compound has a high antiviral activity against Hepatitis C virus, the compound has been developed as a therapeutic agent for hepatitis C (drugs of the Future, 2009, 34(7), 545). Since optically active 1-amino-2-vinylcyclopropane carboxylic acid which is a key intermediate thereof is an important compound as a pharmaceutical intermediate, an industrially convenient production method is demanded.

As the methods for producing optically active 1-amino-2-vinylcyclopropane carboxylic acid, there are known (1) a production method using an asymmetric organic synthesis technique (Organic Research & Development, 2010, 14, 692), (2) a production method by the separation of a diastereomeric salt using an optical resolving agent, and (3) a production method using a biocatalyst. Among these, the production method using an optical resolving technique described in above (2) is currently most widely used since the method has such advantages that optically active 1-amino-2-vinylcyclopropane carboxylic acid is obtained with high stereoselectivity and that special purifying equipment for the separation of a small amount of enantiomer which is by-produced is not required.

As the methods for producing optically active 1-amino-2-vinylcyclopropane carboxylic acid by the separation of a diastereomeric salt using an optical resolving agent, for example, the following methods are reported:

- a method of using Curtius rearrangement after performing optical resolution by reacting racemic vinyl-cyclopropane carboxylic acid with an optically active amine to form a diastereomeric salt (Synthetic Communications, 1994, 24, 2873), and
- a method of reacting a halogenating agent in the presence of a base after reacting racemic 2-vinyl-1-carbamoyl cyclopropane carboxylic acid with an optically active amine to form a diastereomeric salt (International Publication No. WO 2010/041739 (corresponding publication, EP 2345633A)).

However, these methods are difficult to be industrially carried out due to the problems that the Curtius rearrangement reaction is not industrially suitable, that optically active amines are difficult to obtain because multiple steps are required for producing the amines and further that a stoichiometric amount of expensive optically active amines is required for an optical isomer having a target steric structure.

On the other hand, in the production method using a biocatalyst described in the above (3), an optically active compound having high stereoselectivity and high optical purity can be frequently obtained. Further, this method has little effect on the environment because water can be used as a reaction solvent and a biocatalyst corresponding to an industrial scale can be stably and easily obtained by establishing a method for culturing microorganisms which produce an enzyme. For this reason, a production method using a biocatalyst frequently becomes an advantageous process compared with the other methods described above even from an economic viewpoint.

Examples for producing optically active 1-amino-2-vinylcyclopropane carboxylic acid using the biocatalysts reported so far include:

- a method of using Curtius rearrangement by synthesizing a racemic vinylcyclopropane malonic acid diester by the reaction between a malonic acid diester and 1,4-dibromo-2-butene, followed by resolving by a lipase (International

Publication No. WO 2007/088571), and

- a method in which an N-phenylmethylene glycine alkyl ester and trans-1,4-dibromo-2-butene are reacted to synthesize a racemic 1-amino-2-vinylcyclopropane carboxylic acid alkyl ester and the amino group is protected by di-tert-butyl dicarbonate, followed by resolving by an alkali protease (Japanese Patent Laid-Open Publication No. 2010-43124 (corresponding publication, International Publication No. WO 00/09543)).

However, in the former method described in International Publication No. WO 2007/088571, that is, in the method of synthesizing a racemic vinylcyclopropane malonic acid diester by the reaction between a malonic acid diester and 1,4-dibromo-2-butene, followed by resolving by a lipase, since the stereoselectivity is low and the reaction activity is low in resolving by the enzyme, a large amount of enzyme has to be used. Further, this method is not preferable as an industrial production method because a porcine-derived enzyme is required and the Curtius rearrangement reaction is used.

In addition, in the latter method described in Japanese Patent Laid-Open Publication No. 2010-43124 (corresponding publication, International Publication No. WO 00/09543), that is, in the method of synthesizing a racemic 1-amino-2-vinylcyclopropane carboxylic acid alkyl ester by reacting N-phenylmethylene glycine alkyl ester and trans-1,4-dibromo-2-butene and protecting the amino group by di-tert-butyl dicarbonate, followed by resolving by an alkali protease, the stereoselectivity is high, but a tert-butoxycarbonyl group has to be introduced to the amino group before the enzyme reaction. For this reason, at least 2 or more equivalent amount of di-tert-butyl dicarbonate was required based on a target optical isomer and further the amount of enzyme used was required in a large amount.

SUMMARY OF THE INVENTION

The present inventors have succeeded in obtaining a 1-amino-2-vinylcyclopropane carboxylic acid amide having asymmetric carbons at positions 1 and 2 or a salt thereof as a novel precursor compound to 1-amino-2-vinylcyclopropane carboxylic acids. By using this compound, optically active 1-amino-2-vinylcyclopropane carboxylic acids, which are widely used as a raw material for pharmaceutical and agrochemical products, may be produced more inexpensively with high purity and high yield. The present invention is based on these findings.

Consequently, an object of the present invention is to provide a substrate to be subjected to optical resolution, which enables the production of optically active 1-amino-2-vinylcyclopropane carboxylic acids, which are widely used as a raw material for pharmaceutical and agrochemical products and are especially important as an intermediate for therapeutic agents for hepatitis C, inexpensively with high purity and high yield.

More specifically, an object of the present invention is to provide a precursor compound to 1-amino-2-vinylcyclopropane carboxylic acids which satisfy such conditions that it may be subjected to optical resolution, has a molecular weight as low as possible from a view point of atom efficiency, and further may be easily subjected to derivatization required by a technique such as functional group transformation.

That is, the present invention relates to the following compounds and methods.

[1] 1-Amino-2-vinylcyclopropane carboxylic acid amide represented by the following formula (1) or a salt thereof:

[Chemical Formula 1]

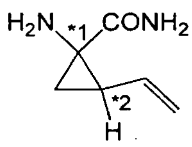

(1)

(wherein, *1 and *2 represent asymmetric carbon).

[2] 1-Amino-2-vinylcyclopropane carboxylic acid amide as described in above [1], which is represented by formula (1).

[3] A salt of 1-amino-2-vinylcyclopropane carboxylic acid amide as described in above [1], which is represented by the following formula (2):

[Chemical Formula 2]

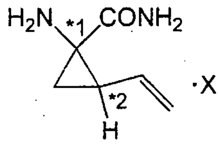

(2)

(wherein, *1 and *2 represent asymmetric carbon and X represents hydrochloric acid, sulfuric acid, nitric acid or acetic acid).

[4] 1-Amino-2-vinylcyclopropane carboxylic acid amide as described in above [1] or [2], which is a (1R, 2S)-isomer or a (1S, 2R)-isomer.

[5] A salt of 1-amino-2-vinylcyclopropane carboxylic acid amide as described in above [3], which is a (1R, 2S)-isomer or a (1S, 2R)-isomer.

[6] A method for producing 1-amino-2-vinylcyclopropane carboxylic acid amide or a salt thereof, comprising hydrolyzing 1-amino-2-vinylcyclopropane carbonitrile represented by the following formula (3) or a salt thereof:

[Chemical Formula 3]

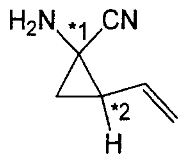

(3)

(wherein, *1 and *2 represent asymmetric carbon)

to obtain 1-amino-2-vinylcyclopropane carboxylic acid amide represented by formula (1) described in above [1] or a salt thereof.

[7] A method for producing 1-amino-2-vinylcyclopropane carboxylic acid or a salt thereof, comprising hydrolyzing 1-amino-2-vinylcyclopropane carboxylic acid amide represented by the following formula (1) or a salt thereof:

[Chemical Formula 4]

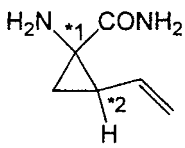

(1)

(wherein *1 and *2 represent asymmetric carbon)

to obtain 1-amino-2-vinylcyclopropane carboxylic acid represented by the following formula (4) or a salt thereof:

[Chemical Formula 5]

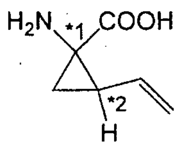

(4)

(wherein, *1 and *2 represent asymmetric carbon).

[8] The method described in above [7], which comprises allowing a microorganism or a processed product thereof having stereoselectively hydrolyzing activity to act on a racemic mixture of 1-amino-2-vinylcyclopropane carboxylic acid amide represented by formula (1) or a salt thereof to generate optically active 1-amino-2-vinylcyclopropane

carboxylic acid represented by formula (4) or a salt thereof.

[9] The method described in above [7] or [8], which comprises allowing a microorganism or a processed product thereof having stereoselectively hydrolyzing activity to act on a mixture containing a (1R, 2S)-isomer and a (1S, 2R)-isomer of 1-amino-2-vinylcyclopropane carboxylic acid amide represented by formula (1) or a salt thereof to generate optically active 1-amino-2-vinylcyclopropane carboxylic acid represented by formula (4).

In addition, the present invention may be rephrased as a compound or a method as described below.

[1'] 1-Amino-2-vinylcyclopropane carboxylic acid amide represented by formula (1).

[2'] A salt of 1-amino-2-vinylcyclopropane carboxylic acid amide represented by formula (2).

[3'] 1-Amino-2-vinylcyclopropane carboxylic acid amide described in above [1'], which is a (1R, 2S)-isomer or a (1S, 2R)-isomer.

[4'] A salt of 1-amino-2-vinylcyclopropane carboxylic acid amide described in above [2'], which is a (1R, 2S)-isomer or a (1S, 2R)-isomer.

[5'] A method for producing 1-amino-2-vinylcyclopropane carboxylic acid amide or a salt thereof, comprising hydrolyzing 1-amino-2-vinylcyclopropane carbonitrile represented by formula (3) or a salt thereof to obtain 1-amino-2-vinylcyclopropane carboxylic acid amide represented by formula (1) or a salt thereof.

[6'] A method for producing 1-amino-2-vinylcyclopropane carboxylic acid or a salt thereof, comprising hydrolyzing 1-amino-2-vinylcyclopropane carboxylic acid amide represented by formula (1) or a salt thereof to obtain 1-amino-2-vinylcyclopropane carboxylic acid represented by formula (4) or a salt thereof.

[7'] A method for producing optically active 1-amino-2-vinylcyclopropane carboxylic acid, which comprises allowing a microorganism or a processed product thereof having stereoselectively hydrolyzing activity to act on a mixture containing a (1R, 2S)-isomer and a (1S, 2R)-isomer of 1-amino-2-vinylcyclopropane carboxylic acid amide represented by formula (1) or a salt thereof to generate 1-amino-2-vinylcyclopropane carboxylic acid represented by formula (4).

## ADVANTAGES OF THE INVENTION

The present invention is capable of easily and efficiently producing optically active 1-amino-2-vinylcyclopropane carboxylic acid amide or a salt thereof, which may be converted in a simple process into optically active 1-amino-2-vinylcyclopropane carboxylic acid that is important as an intermediate for pharmaceutical and agrochemical products, especially, an intermediate for therapeutic agents for hepatitis C.

## DETAILED DESCRIPTION OF THE INVENTION

### 1-Amino-2-Vinylcyclopropane Carboxylic Acid Amide

The compound according to the present invention is a compound represented by formula (1) or a salt thereof, which is 1-amino-2-vinylcyclopropane carboxylic acid amide or a salt thereof useful as an intermediate for pharmaceutical and agrochemical products. In particular, both (1R, 2S)-isomer and (1S, 2R)-isomer of this compound are important as an intermediate for therapeutic agents for hepatitis C.

In a method for producing 1-amino-2-vinyicyclopropane carboxylic acid amide of the present invention, examples of a precursor compound which may be used as a starting material are not particularly limited and include (E)-1-(diphenylmethylene)amino-2-ethenyl cyclopropane carbonitrile which may be synthesized by a method described in J. Org. Chem., 1999, 64 (13), 4712, a compound obtained by treating the carbonitrile with an acid such as hydrochloric acid or a mixture of the two or more. Examples of such compounds include (1R, 2S)-1-amino-2-vinylcyclopropane carbonitrile, (1S, 2R)-1-amino-2-vinylcyclopropane carbonitrile, (1R, 2R)-1-amino-2-vinylcyclopropane carbonitrile and (1S, 2S)-1-amino-2-vinylcyclopropane carbonitrile, and a mixture of the two or more compounds may be used.

In a method for producing 1-amino-2-vinylcyclopropane carboxylic acid amide of the present invention, examples of a method for synthesizing 1-amino-2-vinylcyclopropane carboxylic acid amide from a raw material are not particularly limited and when the starting raw material is 1-amino-2-vinylcyclopropane carbonitrile, 1-amino-2-vinylcyclopropane carboxylic acid amide may be obtained by hydrolyzing using an acid or hydrolyzing using a base.

In the case of hydrolysis using an acid, 1-amino-2-vinylcyclopropane carboxylic acid amide and a salt thereof are generated. Here, examples of an acid used are not particularly limited and include hydrochloric acid, sulfuric acid, hydrofluoric acid, hydrobromic acid, hydroiodic acid, nitric acid, acetic acid, formic acid, phosphoric acid, trifluoroacetic acid, chloroacetic acid, trichloroacetic acid, p-toluene sulfonic acid, methanesulfonic acid, a strong-acid ion exchange resin and a weak-acid ion exchange resin. Among others, it is preferable in terms of operability and economy that acid hydrolysis is performed using hydrochloric acid, sulfuric acid, acetic acid, formic acid, phosphoric acid, p-toluene sulfonic

acid, methanesulfonic acid, a strong-acid ion exchange resin, and the like.

In the case of hydrolysis using a base, 1-amino-2-vinylcyclopropane carboxylic acid amide is generated. Here, examples of a base used are not particularly limited and include a base such as lithium hydroxide, sodium hydroxide and potassium hydroxide.

When 1-amino-2-vinylcyclopropane carboxylic acid amide is obtained as a salt, an acid is used to form the salt. In this case, the kinds of acids are not particularly limited and examples of usable acids include hydrochloric acid, sulfuric acid, hydrofluoric acid, hydrobromic acid, hydroiodic acid, nitric acid, acetic acid, formic acid, phosphoric acid, trifluoroacetic acid, chloroacetic acid, trichloroacetic acid, benzoic acid, citric acid, malonic acid, maleic acid, fumaric acid, butyric acid, isobutyric acid, p-toluene sulfonic acid, methanesulfonic acid, L- or D-tartaric acid, L- or D-lactic acid, L- or D-leucine acid, L- or D-malic acid, L- or D- mandelic acid, o-acetyl-L- or D-mandelic acid, L- or D-aspartic acid, (+)- or (-)-10-camphorsulfonic acid, (+)- or (-)-10-camphorsulfonyl chloride, (+)- or (-)-menthyl chloroformate, L- or D-glutamic acid, (+)- or (-)-camphoric acid, (+)- or (-)-cis-2-benzamidecyclohexanecarboxylic acid, diacetyl-L- or D-tartaric acid, dibenzoyl-L- or D-tartaric acid, di-p-toluoyl-L- or D-tartaric acid, L- or D-pyroglutamic acid, L-or D-quinic acid, (R)- or (S)-propionic acid, (R)- or (S)-2-acetoxypropionic acid, (R)- or (S)-3-acetylthioisobutyric acid, (+)- or (-)-cis-benzamidecyclohexanecarboxylic acid, (R)-or (S)-2-chloropropionic acid, (R)- or (S)-2-bromopropionic acid, 3-chlorobutyric acid, (R)- or (S)-2-chloro-3-methylbutyric acid, (R)- or (S)-2-chloro-4-methylvaleric acid, (R)- or (S)-phenylsuccinic acid, (R)- or (S)-methylsuccinic acid, (R)- or (S)-methylglutaric acid, (R)- or (S)-2,2-dimethylcyclopropanecarboxylic acid, L- or D-citramalic acid, di-p-anysoyl-L- or D-tartaric acid and di-p-benzoyl-L- or D-tartaric acid. Preferred examples of achiral acids include hydrofluoric acid, hydrobromic acid, hydroiodic acid, formic acid, phosphoric acid, trifluoroacetic acid, chloroacetic acid, trichloroacetic acid, benzoic acid, citric acid, malonic acid, maleic acid, fumaric acid, butyric acid, isobutyric acid, p-toluene sulfonic acid, methanesulfonic acid, hydrochloric acid, sulfuric acid, nitric acid and acetic acid, which are easy to use from the viewpoints of easy availability and easy handleability. Especially, hydrochloric acid, sulfuric acid, nitric acid and acetic acid are easy to use because they are inexpensive.

Accordingly, examples of the salt of 1-amino-2-vinylcyclopropane carboxylic acid amide according to the present invention include a salt with the acids exemplified above, preferred examples include a salt of hydrofluoric acid, hydrobromic acid, hydroiodic acid, formic acid, phosphoric acid, trifluoroacetic acid, chloroacetic acid, trichloroacetic acid, benzoic acid, citric acid, malonic acid, maleic acid, fumaric acid, butyric acid, isobutyric acid, p-toluene sulfonic acid, methanesulfonic acid, hydrochloric acid, sulfuric acid, nitric acid and acetic acid, and particularly preferred examples include a salt of hydrochloric acid, sulfuric acid, nitric acid and acetic acid.

When 1-amino-2-vinylcyclopropane carboxylic acid amide is obtained by hydrolysis, an acid for separately forming a salt is added after hydrolysis. An acid for forming a salt may be added after neutralization.

In the acid hydrolysis or alkali hydrolysis of 1-amino-2-vinylcyclopropane carbonitrile of the present invention, 0.001 to 50 moles and preferably 0.01 to 10 moles of an acid or a base is used based on 1 mole of 1-amino-2-vinylcyclopropane carbonitrile.

The hydrolysis reaction of 1-amino-2-vinylcyclopropane carbonitrile of the present invention may be carried out by stirring at -20 to 150°C and preferably at -5 to 50°C for 0.1 to 24 hour. In this case, desired conditions are such that the resulting 1-amino-2-vinylcyclopropane carboxylic acid amide is no further hydrolysed to form 1-amino-2-vinylcyclopropane carboxylic acid

In addition, the hydrolysis reaction of 1-amino-2-vinylcyclopropane carbonitrile of the present invention may be carried out under the coexistence of water, an organic solvent (for example, alcohols such as methanol, ethanol, n-propanol, isopropanol, n-butanol and isobutanol; ethers such as tetrahydrofuran, diethyl ether, 1,4-dioxane, tert-butylmethyl ether, tert-butylethyl ether, diisopropyl ethyl ether and cyclopentyl methyl ether; esters such as methyl acetate, ethyl acetate, propyl acetate and isopropyl acetate; halogenated hydrocarbons such as dichloromethane, chloroform and 1,4-dichloroethane), an additive for promoting hydrolysis (for example, ketones such as acetone, methylethyl ketone, diethyl ketone, methylisobutyl ketone, diethyl ketone, isopropyl ethyl ketone, methylisobutyl ketone, cyclopentane and cyclohexane) or the like.

1-Amino-2-vinylcyclopropane carboxylic acid or a salt thereof may be obtained by hydrolyzing 1-amino-2-vinylcyclopropane carboxylic acid amide or a salt thereof of the present invention. Examples of a method for hydrolysis are not particularly limited and include a method of using a biocatalyst and a method of using an acid or a base.

In the case of hydrolysis using an acid, 1-amino-2-vinylcyclopropane carboxylic acid and a salt thereof are generated. Examples of an acid used are not particularly limited and include sulfuric acid, hydrochloric acid, hydrofluoric acid, hydrobromic acid, hydroiodic acid, nitric acid, acetic acid, formic acid, phosphoric acid, trifluoroacetic acid, chloroacetic acid, trichloroacetic acid, p-toluene sulfonic acid, methanesulfonic acid and a strong-acid ion exchange resin, which may be used in combination with two or more kinds. In the case of hydrolysis using a base, 1-amino-2-vinylcyclopropane carboxylic acid is generated. Examples of a base used are not particularly limited, and for example, alkali hydrolysis using a base such as lithium hydroxide, sodium hydroxide and potassium hydroxide may be performed. Especially, it is preferable from the viewpoints of operability and economy that 1-amino-2-vinylcyclopropane carboxylic acid amide is hydrolyzed using an acid such as sulfuric acid, hydrochloric acid, acetic acid, formic acid, phosphoric acid,

p-toluene sulfonic acid and methanesulfonic acid, or using a base such as lithium hydroxide, sodium hydroxide and potassium hydroxide, because 1-amino-2-vinylcyclopropane carboxylic acid is obtained easily and inexpensively.

When 1-amino-2-vinylcyclopropane carboxylic acid is obtained as a salt, an acid is used for forming the salt. Examples of the kind of the acid are not particularly limited and include hydrochloric acid, sulfuric acid, hydrofluoric acid, hydrobromic acid, hydroiodic acid, nitric acid, acetic acid, formic acid, phosphoric acid, trifluoroacetic acid, chloroacetic acid, trichloroacetic acid, benzoic acid, citric acid, malonic acid, maleic acid, fumaric acid, butyric acid, isobutyric acid, p-toluene sulfonic acid, methanesulfonic acid, L- or D-tartaric acid, L- or D-lactic acid, L- or D-leucine acid, L- or D- malic acid, L- or D-mandelic acid, o-acetyl-L- or D-mandelic acid, L- or D-aspartic acid, (+)- or (-)-10-camphorsulfonic acid, (+)- or (-)-10-camphorsulfonyl chloride, (+)- or (-)-menthyl chloroformate, L- or D-glutamic acid, (+)- or (-)-camphoric acid, (+)- or (-)-cis-2-benzamidecyclohexanecarboxylic acid, diacetyl-L- or D-tartaric acid, dibenzoyl-L- or D-tartaric acid, di-p-toluoyl-L- or D-tartaric acid, L- or D-pyroglutamic acid, L-or D-quinic acid, (R)- or (S)-propionic acid, (R)- or (S)-2-acetoxypropionic acid, (R)- or (S)-3-acetylthioisobutyric acid, (+)- or (-)-cis-benzamidecyclohexanecarboxylic acid, (R)-or (S)-2-chloropropionic acid, (R)- or (S)-2-bromopropionic acid, 3-chlorobutyric acid, (R)- or (S)-2-chloro-3-methylbutyric acid, (R)- or (S)-2-chloro-4-methylvaleric acid, (R)- or (S)-phenylsuccinic acid, (R)- or (S)-methylsuccinic acid, (R)- or (S)-methylglutaric acid, (R)- or (S)-2,2-dimethylcyclopropanecarboxylic acid, L- or D-citramalic acid, di-p-anysoyl-L- or D-tartaric acid and di-p-benzoyl-L- or D-tartaric acid. Preferred examples of achiral acids include hydrofluoric acid, hydrobromic acid, hydroiodic acid, formic acid, phosphoric acid, trifluoroacetic acid, chloroacetic acid, trichloroacetic acid, benzoic acid, citric acid, malonic acid, maleic acid, fumaric acid, butyric acid, isobutyric acid, p-toluene sulfonic acid, methanesulfonic acid, hydrochloric acid, sulfuric acid, nitric acid and acetic acid, which are easy to use from the viewpoints of easy availability and easy handleability. Especially, hydrochloric acid, sulfuric acid, nitric acid and acetic acid are easy to use because they are inexpensive.

Accordingly, examples of a salt of 1-amino-2-vinylcyclopropane carboxylic acid amide according to the present invention include a salt with the acids exemplified above, preferred examples of the acids include hydrofluoric acid, hydrobromic acid, hydroiodic acid, formic acid, phosphoric acid, trifluoroacetic acid, chloroacetic acid, trichloroacetic acid, benzoic acid, citric acid, malonic acid, maleic acid, fumaric acid, butyric acid, isobutyric acid, p-toluene sulfonic acid, methanesulfonic acid, hydrochloric acid, sulfuric acid, nitric acid and acetic acid, and particularly preferred examples include a salt of hydrochloric acid, sulfuric acid, nitric acid and acetic acid.

When 1-amino-2-vinylcyclopropane carboxylic acid is obtained by hydrolysis, an acid for separately forming a salt is added after hydrolysis. An acid for forming a salt may be added after neutralization.

In the acid hydrolysis or alkali hydrolysis of 1-amino-2-vinylcyclopropane carboxylic acid amide of the present invention, 0.001 to 50 moles and preferably 0.01 to 10 moles of an acid or a base is used based on 1 mole of 1-amino-2-vinylcyclopropane carboxylic acid amide.

The hydrolysis reaction of 1-amino-2-vinylcyclopropane carboxylic acid amide of the present invention may be carried out chemically by stirring at 0 to 180°C and preferably at 55 to 100°C for 0.1 to 72 hours. In this case, when the reaction is carried out under the same reagent conditions as those in the case where 1-amino-2-vinylcyclopropane carbonitrile is chemically hydrolyzed, the reaction is carried out at a temperature higher than the temperature at which 1-amino-2-vinylcyclopropane carbonitrile is hydrolyzed, or may be carried out by elongating the reaction time.

In addition, the hydrolysis reaction of 1-amino-2-vinylcyclopropane carboxylic acid amide of the present invention may be carried out under the coexistence of water, an organic solvent (for example, alcohols such as methanol, ethanol, n-propanol, isopropanol, n-butanol and isobutanol; ethers such as tetrahydrofuran, diethyl ether, 1,4-dioxane, tert-butylmethyl ether, tert-butylethyl ether, diisopropyl ethyl ether and cyclopentyl methyl ether; esters such as methyl acetate, ethyl acetate, propyl aqcetate and isopropyl acetate; halogenated hydrocarbons such as dichloromethane, chloroform and 1,4-dichloroethane) or the like.

Further, 1-amino-2-vinylcyclopropane carboxylic acid amide and a salt thereof obtained by the present invention may be used as pharmaceutical and agrochemical raw materials obtained after derivatization.

Examples of a compound derived from 1-amino-2-vinylcyclopropane carboxylic acid amide or a salt thereof include 1-amino-2-vinylcyclopropane carboxylic acid, 1-amino-2-ethylcyclopropane carboxylic acid amide, 1-amino-N-(cyclopropylsulfonyl)-2-vinylcyclopropane carboxylic acid amide, 1-methylamino-2-vinylcyclopropane carboxylic acid amide, 1-ethylamino-2-vinylcyclopropane carboxylic acid amide, 1-propylamino-2-vinylcyclopropane carboxylic acid amide, 1-isopropylamino-2-vinylcyclopropane carboxylic acid amide, 1-amino-2-vinylcyclopropane carbohydrazide, 1-amino-2-vinylcyclopropane carbothioamide, 1-amino-2-formylcyclopropane carboxylic acid amide and 1-amino-2-(oxiran-2-yl)cyclopropane carboxylic acid amide.

1-Amino-2-vinylcyclopropane carboxylic acid amide or a salt thereof may be optically resolved using a biocatalyst of a microorganism having stereoselectively hydrolyzing activity of an optically active carboxylic acid amide.

Specific examples of the biocatalyst include, but not limited to, a microorganism belonging to Xanthobacter sp., Serratia sp., Chromobacterium sp., Protaminobacter sp., Pseudomonas sp., Microbaterium sp., and the like or a processded product of the microorganism. More specific examples include Protaminobacter alboflavus ATCC8458, Xanthobacter autotrophicus DMS597 and Chromobacterium iodium IFO3558. In addition, even in either case of a mutant strain

derived from these microorganisms by artificial mutation means or a recombinant strain derived from these microorganisms by a genetic engineering technique such as a cell fusion method or a genetic recombination method, a strain having the above ability may be used in the present invention. The cultivation of these microorganisms is generally carried out using a culture medium prepared by incorporating assimilable carbon sources, nitrogen sources, inorganic salts essential for each microorganism, nutrients or the like. The pH during cultivation is preferably in the range of 4 to 10 and the temperature is preferably 20 to 50°C. The cultivation is carried out aerobically for approximately one day to one week. The microorganisms cultivated in this manner are used as a microorganism or a processed product of the microorganism, for example, a culture solution, a separated microorganism, a fractured microorganism or further a purified enzyme. In addition, a microorganism or an enzyme may be used after immobilization according to a conventional method.

In a method of subjecting (E)-1-amino-2-vinylcyclopropane carboxylic acid amide or a salt thereof to stereoselective amide hydrolysis reaction by a biocatalyst, optical resolution is possible without performing a special modification, for example, special modification to an amino group, compared with the optical resolution by an ester hydrolysis enzyme such as lipase. Therefore, the method is preferred in that the synthesis step of a reaction substrate may be simplified, and the method is preferable in terms of atom efficiency because the method has an effect of reducing the weight of an unnecessary optical isomer. In addition, since the water solubility of the reaction substrate is maintained because no special modification is added to a substituent having high polarity and the amount used of an amphiphilic organic solvent, which is made to coexist with water in order to dissolve the reaction substrate, may be reduced, the method is useful in terms of safety and economy. Further, when 1-amino-2-vinylcyclopropane carboxylic acid amide is hydrolyzed, a mixture of a (1R, 2S)-isomer and a (1S, 2R) isomer may be used.

Optically active 1-amino-2-vinylcyclopropane carboxylic acid amide and a salt thereof of the present invention may also be converted into a derivative useful as pharmaceutical and agrochemical intermediates by chemical modification of the carboxylic acid amide site. Examples of the chemical modification include an N-alkylation reaction, an acid hydrazination reaction, an ozonolysis reaction, an imidization reaction, a thioamidation reaction, an oxidation reaction and a catalytic hydrogenation reaction, which may be carried out to obtain useful intermediates for pharmaceutical and agrochemical products.

EXAMPLES

Hereinafter, the present invention will be specifically explained with reference to Examples, but the present invention is not limited to these Examples.

Measurement of Optical Purity

In the present Examples, the optical purity of 1-amino-2-vinylcyclopropane carboxylic acid amide and 1-amino-2-vinylcyclopropane carboxylic acid was determined by HPLC analysis. Further, the following conditions were used in these analyses.

- Column: SUMICHIRAL (Registered Trademark) OA-5000 (4.6 x 50 mm, 5 μm)
- Mobile Phase: 1 mM, $CuSO_4 \cdot 5H_2O$
- Flow Rate: 1 mL/min.
- Detector: UV 210 nm

Reference Example 1:

A solution was prepared by dissolving 280 mg (0.5 mmol) of bis(dibenzylideneacetone)palladium (0) and 263 mg (1 mmol) of triphenylphosphine in 40 mL of tetrahydrofuran under a nitrogen atmosphere, followed by stirring at room temperature for 10 minutes. Into the solution was added 20 mL of a tetrahydrofuran solution of 1.25 g (10 mmol) of 1,4-dichlorobutadiene. After the color of the solution turned orange, 20 mL of a tetrahydrofuran solution of 2.52 g (12 mmol) of N-(diphenylmethylene)aminoacetonitrile and 1.07 g (20 mmol) of 45% sodium hydride (in oil) were added. The resulting reaction solution was stirred for 15 minutes, followed by filtering on celite and the resulting organic layer was washed with 30 mL of water and 30 mL of saturated saline. After the organic layer was concentrated under reduced pressure, the residue was refined by silica gel chromatography using 20% diethyl ether/petroleum ether as an eluent to obtain the refined (E)-1-(diphenylmethylene)amino-2-ethenylcyclopropane carbonitrile as a transparent yellow oil (2.32 g, yield: 71%).
$^1$H NMR ($CDCl_3$) 1.69 (dd, J=8.2, 5.6 Hz, 1H), 1.94 (dd, J=8.2, 5.6 Hz, 1H), 2.37 (ddd, J=8.2, 8.2, 7.1 Hz, 1H), 5.24-5.36 (m, 2H), 5.55 (ddd, J=15.3, 8.4, 7.1 Hz, 1H), 7.12-7.65 (m, 10H)

Example 1:

A solution was prepared by dissolving 1.39 g (5.1 mmol) of (E)-1-(diphenylmethylene)amino-2-ethenylcyclopropane carbonitrile obtained in the same manner as in Reference Example 1 in 5 mL of toluene, followed by dropwise addition of 4.25 mL (51 mmol) of a 12 N hydrochloric acid aqueous solution at 0°C. The solution was stirred at the temperature (0°C) for 30 minutes, followed by removal of an organic layer by liquid separation. The aqueous layer was stirred at 40°C for 2 hours, followed by the addition of 4.25 mL of water and concentration under reduce pressure to obtain a hydrochloride of a mixture of (1R, 2S)-isomer and (1S, 2R)-isomer of 1-amino-2-vinylcyclopropane carboxylic acid amide as ocher crystals (813 mg, yield: 98%).
$^{1}H$ NMR ($D_2O$) $\delta$ 1.61 (dd, J=10.3, 8.0 Hz, 1H), 1.72 (t, J=8.0 Hz, 1H), 2.30 (dd, 17.2, 8.0 Hz, 1H), 5.21 (d, J=10.3 Hz, 1H), 5.29 (d, J=17.2 Hz, 1H), 5.63 (ddd, J=17.2, 10.3, 6.8 Hz, 1H) $^{13}C$ NMR ($D_2O$) $\delta$ 16.7, 28.8, 41.0, 121.2, 131.2, 170.9

Example 2:

A solution was prepared by dissolving 1.85 g (6.8 mmol) of (E)-1-(diphenylmethylene)amino-2-ethenylcyclopropane carbonitrile obtained in the same manner as in Reference Example 1 in 7 mL of toluene, followed by dropwise addition of 5.65 mL (33.9 mmol) of a 6 N hydrochloric acid aqueous solution at 0°C. The solution was stirred at the temperature (0°C) for 30 minutes, followed by removal of an organic layer by liquid separation. The residue obtained by concentrating the resulting aqueous layer under reduced pressure was dissolved in a mixed solution of 13.7 mL (20.5 mmol) of a 1.5 N sodium hydroxide aqueous solution and 5.7 mL of acetone, followed by stirring at room temperature for one hour. The resulting solution was neutralized with a 10% aqueous solution of $NH_4Cl$, followed by concentration under reduced pressure. The residue was suspended in 10 mL of methanol to separate insoluble matters by filtration. The filtrate was distilled off under reduced pressure to obtain a mixture of (1R, 2S)-isomer and (1S, 2R)-isomer of a 1-amino-2-vinylcyclopropane carboxylic acid amide as ocher crystals (0.70 g, yield: 82%).

Example 3:

A culture medium having a composition described in the following Table 1 was prepared and 200 mL of the culture medium was placed in a 1 L Erlenmeyer flask. After sterilization, the flask was inoculated with Xanthobacter autotrophicus DSM597 (obtained from RIKEN BioResource Center) and shaking cultivation was carried out at 30°C for 72 hours.

[Table 1]
Culture Medium Composition (pH: 7.0)

| | |
|---|---|
| Trypsin | 10 g |
| Yeast Extract | 5 g |
| NaCl | 5 g |
| Water | 1 L |

Subsequently, from the culture solution, a concentrated microorganism corresponding to 0.5 g of a dried microorganism was obtained by centrifugation.
A solution was prepared by dissolving 1.0 g (7.93 mol) of a mixture (racemic mixture) of (1R, 2S)-isomer and (1S, 2R)-isomer of 1-amino-2 vinylcyclopropane carboxylic acid amide obtained in the same manner as in Example 2 in 100 mL of water. The solution was placed in 300 mL flask and the concentrated microorganism corresponding to 0.5.g of a dried microorganism was added, followed by stirring at 30°C for 5 hours to perform hydrolysis reaction. After the reaction, when the supernatant obtained by removing the microorganism from the reaction solution by centrifugation was analyzed by HPLC, the conversion of the reaction was 25% and the resulting (1S, 2R)-1-amino-2-vinyl-cyclopropane carboxylic acid has an optical purity of more than 99% e.e.
In addition, the above conversion may be calculated as follows using the area value of each isomer obtained by HPLC measurement.

$$- \text{Conversion} = [(\text{Area value of (1R, 2S) isomer of the above amide} - \text{Area value of (1S, 2R) isomer of the above amide}) \times 100]/\text{Area value of (1R, 2S) isomer of the above amide}$$

(Here, since the "area value of the (1R, 2S)-isomer of amide" in the above expression may be said to correspond to a value of the "(1S, 2R)-isomer of amide" before the hydrolysis reaction because the (1R, 2S)-isomer of amide and the (1S, 2R)-isomer of amide are considered to be present in the same amount in a racemic mixture, the conversion was calculated by the above expression.)

Example 4:

A solution was prepared by dissolving 50 mg (0.31 mmol) of a hydrochloride of a mixture of (1R, 2S)-isomer and (1S, 2R)-isomer of 1-amino-2-vinylcyclopropane carboxylic acid amide obtained in Example 1 in 0.62 mL (0.93 mmol) of a 1.5 N sodium hydroxide aqueous solution, followed by heating and stirring at 70°C. 1-Amino-2-vinylcyclopropane carboxylic acid was generated at a conversion of 83% of 1-amino-2-vinylcyclopropane carboxylic acid amide after the reaction time of 3 hours.

In addition, the conversion in Example 4 may be calculated by the following expression using the area value of each component obtained by HPLC measurement under the following conditions.

$$\text{Conversion} = [(\text{Amide area before the reaction} - \text{Amide area after the reaction})/\text{Amide area before the reaction}] \times 100$$

[HPLC Analysis]

- Column: Inertsil ODS-3 (4.0 x 250 mm, 5 μm)
- Mobile Phase: MeCN: 30 mM Sodium dihydrogen phosphate = 60: 40
- Flow Rate: 1 mL/min.
- Detector: UV 210 nm

INDUSTRIAL APPLICABILITY

1-Amino-2-vinylcyclopropane carboxylic acid amide and a salt thereof obtained by the present invention are very useful as production intermediates for pharmaceutical and agrochemical products.

**Claims**

**1.** 1-Amino-2-vinylcyclopropane carboxylic acid amide represented by the following formula (1) or a salt thereof:

[Chemical Formula 1]

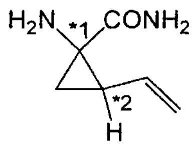

(1)

(wherein, *1 and *2 represent asymmetric carbon).

**2.** 1-Amino-2-vinylcyclopropane carboxylic acid amide according to claim 1, which is represented by formula (1).

**3.** A salt of 1-Amino-2-vinylcyclopropane carboxylic acid amide according to claim 1, which is represented by the

following formula (2):

[Chemical Formula 2]

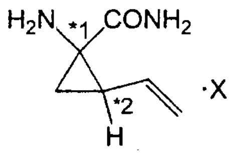

(2)

(wherein, *1 and *2 represent asymmetric carbon and X represents hydrochloric acid, sulfuric acid, nitric acid or acetic acid).

**4.** 1-Amino-2-vinylcyclopropane carboxylic acid amide according to claim 1 or 2, which is a (1R, 2S)-isomer or a (1S, 2R)-isomer.

**5.** A salt of 1-Amino-2-vinylcyclopropane carboxylic acid amide according to claim 3, which is a (1R, 2S)-isomer or a (1S, 2R)-isomer.

**6.** A method for producing 1-amino-2-vinylcyclopropane carboxylic acid amide or a salt thereof, comprising hydrolyzing 1-amino-2-vinylcyclopropane carbonitrile represented by the following formula (3) or a salt thereof:

[Chemical Formula 3]

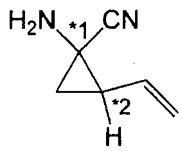

(3)

(wherein, *1 and *2 represent asymmetric carbon.) to obtain 1-amino-2-vinylcyclopropane carboxylic acid amide represented by formula (1) according to claim 1 or a salt thereof.

**7.** A method for producing 1-amino-2-vinylcyclopropane carboxylic acid or a salt thereof, comprising hydrolyzing 1-amino-2-vinylcyclopropane carboxylic acid amide represented by the following formula (1) or a salt thereof:

[Chemical Formula 4]

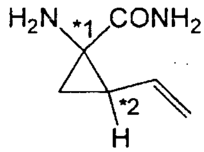

(1)

(wherein *1 and *2 represent asymmetric carbon.) to obtain 1-amino-2-vinylcyclopropane carboxylic acid represented by the following formula (4) or a salt thereof:

[Chemical Formula 5]

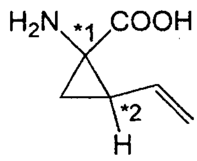

(4)

(wherein, *1 and *2 represent asymmetric carbon).

**8.** The method according to claim 7, which comprises allowing a microorganism or a processed product of the microorganism having stereoselectively hydrolyzing activity to act on a racemic mixture of 1-amino-2-vinylcyclopropane carboxylic acid amide represented by formula (1) or a salt thereof to generate optically active 1-amino-2-vinylcyclopropane carboxylic acid represented by formula (4) or a salt thereof.

**9.** The method according to claim 7 or 8, which comprises allowing a microorganism or a processed product of the microorganism having stereoselectively hydrolyzing activity to act on a mixture containing a (1R, 2S)-isomer and a (1S, 2R)-isomer of 1-amino-2-vinylcyclopropane carboxylic acid amide represented by formula (1) or of a salt thereof to generate optically active 1-amino-2-vinylcyclopropane carboxylic acid represented by formula (4).

**INTERNATIONAL SEARCH REPORT**

International application No.
PCT/JP2012/065452

A. CLASSIFICATION OF SUBJECT MATTER
*C07C237/24*(2006.01)i, *C07C227/18*(2006.01)i, *C07C227/30*(2006.01)i, *C07C229/48*(2006.01)i, *C07C231/06*(2006.01)i, *C07C231/16*(2006.01)i, *C12P13/02*(2006.01)i, *C07B53/00*(2006.01)n

According to International Patent Classification (IPC) or to both national classification and IPC

B. FIELDS SEARCHED

Minimum documentation searched (classification system followed by classification symbols)
C07C237/24, C07C227/18, C07C227/30, C07C229/48, C07C231/06, C07C231/16, C12P13/02, C07B53/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2008/070358 A2 (Phenomix Corp.), 12 June 2008 (12.06.2008), claims 1, 34; schemes 3, 4, 10 to 12 (Family: none) | 1-9 |
| A | US 2011/0135599 A1 (Gilead Sciences, Inc.), 09 June 2011 (09.06.2011), pages 72, 75 (Family: none) | 1-9 |
| A | JP 2009-536158 A (Novartis AG.), 08 October 2009 (08.10.2009), claims 17, 18; tables A, B; paragraph [0250] & US 2007/0265281 A1 & WO 2007/121125 A2 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 July, 2012 (27.07.12) | 07 August, 2012 (07.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/065452

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-516981 A (Japan Tobacco Inc.), 28 June 2007 (28.06.2007), paragraph [0155] & US 2005/0222146 A1 & WO 2005/058808 A1 | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011137246 A
- WO 2010041739 A
- EP 2345633 A
- WO 2007088571 A
- JP 2010043124 A
- WO 0009543 A

### Non-patent literature cited in the description

- *drugs of the Future,* 2009, vol. 34 (7), 545
- *Synthetic Communications,* 1994, vol. 24, 2873
- *J. Org. Chem.,* 1999, vol. 64 (13), 4712